# EUROPEAN PATENT SPECIFICATION

(11) **EP 0 748 611 B1**
(45) Date of publication and mention of the grant of the patent: **26.03.2003**
(21) Application number: 96830327.1
(22) Date of filing: 06.06.1996
(51) Int. Cl.: A61B 5/0452, A61B 5/044, A61G 15/14

(54) **Heart function monitoring system applied to dental apparatus**
Dentalgerät mit einer Vorrichtung zur Herzfunktionsüberwachung
Appareil dentaire avec un dispositif de surveillance de la fonction cardiaque

(30) Priority: 08.06.1995 IT BO950291
(43) Date of publication of application: 18.12.1996
(73) Proprietor: CASTELLINI S.p.A., I-40013 Castel Maggiore (Bologna) (IT)
(72) Inventor: Castellini, Franco, 40123 Bologna (IT)
(74) Representative: Lanzoni, Luciano

(56) References cited:
- EP-A- 0 584 439
- WO-A-87/06447
- FR-A- 2 666 977
- US-A- 4 589 420
- US-A- 4 938 228

## Description

The present invention relates to a system for monitoring heart functions which may be fitted to dental apparatus such as dental units.

The enormous progress made in the application of hardware and software technology to dentistry has enabled modern dental surgeries to make increasing use of dental units equipped with high-technology systems and accessories.

Evidently, this extraordinary technological development has been influenced by the ever increasing degree of specialization of dental surgeons whose work now more than ever before includes sophisticated surgical operations.

Surgical intervention may be complex, lengthy and critical for the patient and the dental surgeon must be in a position to monitor the state of the patient's primary functions during an operation, especially if the operation requires large doses of anaesthetic in the oral cavity for 'an extended length of time.

In this regard, the Applicant has designed and produced a dental unit with a built-in electrocardiograph having a viewer for displaying the electrocardiographic signal obtained through sensors applied to parts of the patient's body (see also patent publication EP-584.439). This solution allows the surgeon to monitor the patient's heart functions from the beginning to the end of the operation without requiring any other specific equipment in the immediate vicinity of the working area.

An apparatus for ECG rhythm analysis is known from document US-4-589-420 which discloses an ECG lead, attached to a patient, to send signals to an A/D converter and to a microprocessor. The apparatus further comprises a portion of memory capable of storing continuos digital samples of signals, matching them with a template formed during a learn mode and creating an alarm if the incoming signals are classified as abnormal. The signals stored in memory are transferred to a display upon command by the attending physician.
Moreover, it is also known from document WO-87-06447 an electrocardiograph having input terminal means, an A/D converter for converting electrical signals applied to the input terminal means into digital signals, a memory device for storing the digital signals and display means for graphically displaying the digital signals.

The above solution does, however, unduly encumber the dental unit since the viewer (which performs this function only) subtracts space from the handpiece and instrument tablet. In addition, part of the electronic equipment connected to the electrocardiograph is separate from the electronic and hydraulic assemblies forming part of the dental unit.

Moreover, the solution disclosed in the abovementioned publication EP-584 439, although it has positively contributed to the technological development of dental units, is nevertheless a "passive" tool in that the pulsation of the heart is simply measured by the apparatus (and in some cases recorded on paper) whilst it is left to the surgeon to check the correctness of the measurement. That means the surgeon or the surgeon's assistant has to interrupt the operation at regular intervals to check the signal, read the graphic record and detect anomalies, if any.

For this reason, the Applicant has devised a solution whereby the heart function monitoring system derived from the reading of the electrocardiograph can be incorporated into the control system of the dental engine so that monitoring of the heart functions during the operation, especially in the event of pathological conditions, becomes an "active" part of the dental unit capable of generating an alarm signal to alert the surgeon when an anomalous condition arises.

The characteristics of the invention are laid out in the claims below and the advantages of the disclosure are apparent from the detailed description which follows, with reference to the accompanying drawings, which illustrate a preferred embodiment of the invention and in which:
- Figure 1 is a perspective side view of a dental unit equipped with the heart function monitoring system disclosed by the present invention;
- Figure 2 is a scaled up, schematic front view of detail A of Fig. 1, namely a viewer for displaying an electrocardiographic signal;
- Figure 3 is a scaled up, partial perspective view of detail B of Fig. 1, showing the area where the sensors that measure the electrocardiographic signal lead out of the dental unit and the points on the patient's body where the sensors should be applied;
- Figure 4 is a block diagram showing the components making up the system disclosed;
- Figure 5 shows a display page with a submenu envisaged by the system disclosed;
- Figure 6 is a block diagram showing the steps in the processing effected by the system.

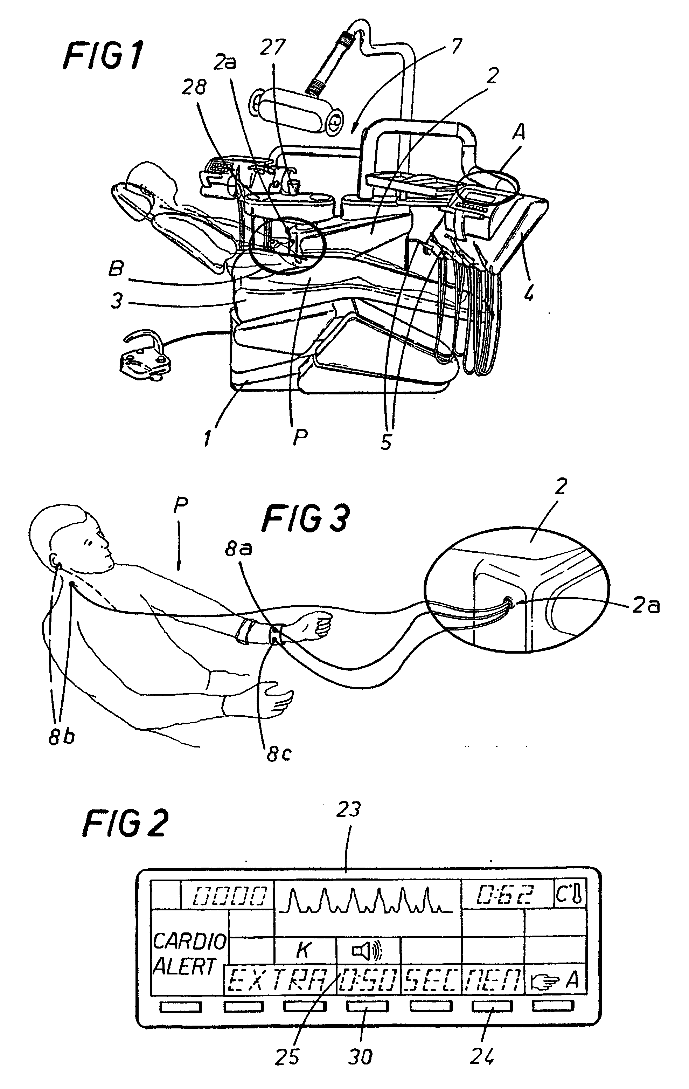

In accordance with the accompanying drawings, and in particular Fig.1, the heart function monitoring system disclosed herein is applied to dental apparatus such as dental units.

These dental units 7, as shown in Fig. 1, basically include a base 1 supporting a first column 2, placed beside a chair 3 that is adjustable in height and angle, and a first main table or tablet 4 to hold dental instruments 5 such as basic handpieces (for example, a micromotor, a turbine, a syringe and a scaler, illustrated schematically in the drawing).

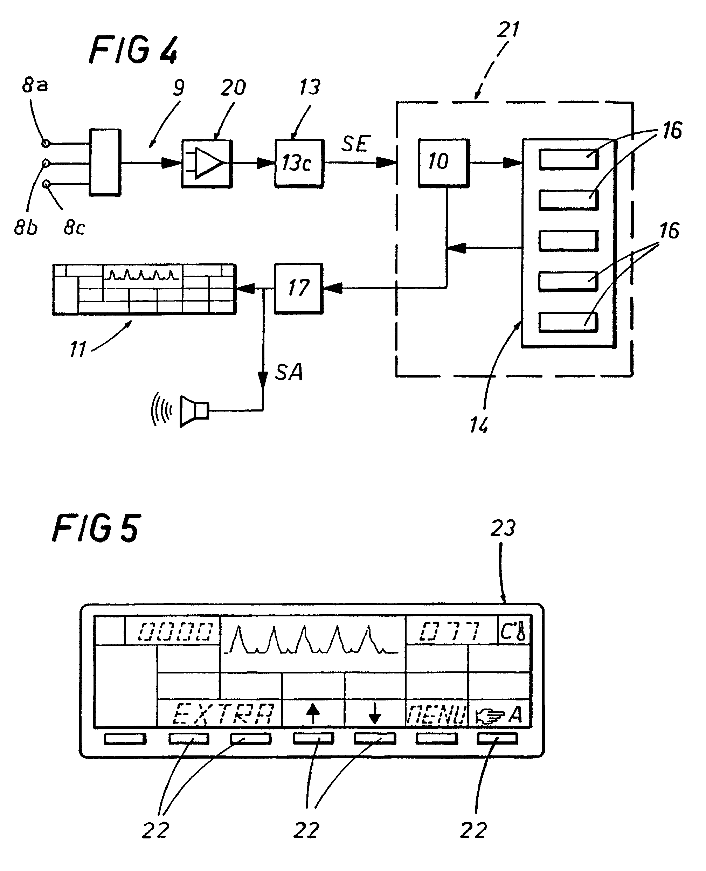

In addition to these, the dental unit 7 also envisages a plurality of sensors 8 which form the contact terminals of an electrocardiograph 9 and which are housed in, and feed out of, the column 2, for example through a hole 2a (see Fig. 3). In the preferred embodiment, there are three sensors (labelled 8a, 8b and 8c) which can be applied to specific parts of the body of the patient P in order to detect an electrocardiographic signal, labelled SE in Fig.4.

In practice, the three sensors 8a, 8b and 8c (which constitute the minimum configuration required to measure basic cardiac activity), consist of two input contacts (those labelled 8a and 8b constituting the main detecting elements) which actually measure the electrocardiographic signal SE, whilst the third sensor 8c is the neutral contact necessary to reduce line noise interfering with the measured signal. In the preferred embodiment, again as illustrated in Fig. 3, where the requirement of electrocardiographic measurement that the input sensors be placed on opposite sides of the heart has to be adapted to the patient's position and attitude during a dental treatment session, the input sensors 8a and 8b are applied to the left wrist of the patient P and to the right-hand side of the patient's neck (or to the lobe of the right ear, i.e. on the side of the body opposite that where the first sensor is applied). The third, neutral, sensor 8c, on the other hand, is applied near the first sensor, in this case, to the left wrist.

To control the dental unit 7 there is envisaged a monitoring unit or microprocessor 10, which also controls a viewer 11 (see Fig. 2) for continuously displaying the operating function data and the accessory function data of the aforestated instruments 5. The viewer 11 consists preferably of a Vacuum Fluorescent Display (VFD) on which the microprocessor 10 can display the monitored functions of the dental unit (preset handpiece speed, temperature of fluids, disinfection or sterilization cycles, cycle time settings, and so on).

In addition to these functions, the monitoring system constituted by the microprocessor 10 is equipped (see Fig. 4) with means 13 for the analog/digital conversion of an electrocardiographic signal SE, the input of the said means being connected to sensors 8 and the output to the microprocessor 10 so that the latter can carry out the necessary digital processing. The electrocardiographic signal SE may also be stored by storage means 14 and displayed by the viewer 11 (as described in more detail below).

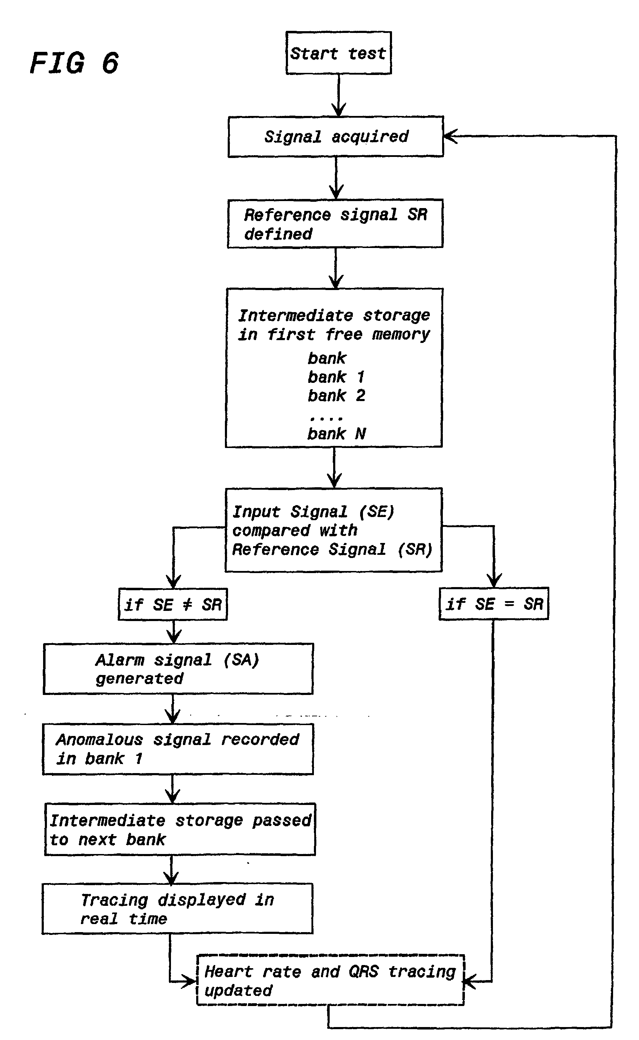

To make the configuration of the heart function monitoring system "active", the present invention envisages (see Fig. 6) means for comparing the processed electrocardiographic input signal SE with a previously stored reference signal SR: this comparison makes it possible to generate an alarm signal, labelled SA and a corresponding display on the viewer 11 if the signal SE is anomalous or differs in any way from the reference signal SR. In other words, the alarm is triggered only when a pathologically incorrect event is detected in the tracing of the signal SE.

Looking more closely at the technical details, the conversion means 13 may consist, for example, of an analog/digital converter 13c whose input is connected to an amplifier 20 of the electrocardiographic signal from the sensors 8 and whose output is connected to the microprocessor 10. The microprocessor 10, which also forms the comparator means and which is an integral part of a processing and digital storage block 21 including the aforesaid storage means 14, processes the reference signal SR when the electrocardiograph 9 on the dental unit 7 is switched on and updates the reference signal, at least as regards the heart rate data item, at regular, preset intervals.

The aforesaid storage means 14 consist of an independent unit connected to the microprocessor 10 and, preferably, the means 14 comprise a plurality of N memory banks 16 which can record a plurality of single tracings of the processed electrocardiographic signal SE made in defined units of time depending on the storage capacity of each bank 16. The tracings are stored sequentially, bank after bank, in first in first out fashion, in such a way that the signals indicating an anomalous event are always the most recent.

The memory therefore comprises N banks in which processed portions of tracing SE with anomalous events are recorded one after the other in first in first out fashion: in other words, the tracing of the measured signal SE passes to the first free bank without being either stored or displayed ("intermediate storage"). When an anomalous event occurs, the portion of tracing with the anomalous event is recorded on that bank, whilst the normal signal SE is passed to the next bank. When the N memory banks are all full, the next anomalous tracing is recorded on the first memory bank, that is to say, it overwrites the oldest anomalous event.

The processing unit 21 is capable of displaying the contents of any the said memory banks 16 on the viewer 11 when the surgeon requests it using appropriate selection means 22 which act directly on the microprocessor 10. The means 22 may be constituted by pushbuttons located on a console 23 placed beside the viewer 11.

The processing unit 21 (see Fig. 4) is also connected to a control unit 17 of the viewer 11 capable of activating the display page showing the electrocardiographic signal SE measured in real time and disabling the display pages active at that moment which show data related to the instruments 5: in other words, when an anomalous event is detected in the tracing of the signal SE (the event, as stated earlier, being recorded on the first available memory bank 16) the alarm signal SA, preferably audible, is triggered and the control unit 17 automatically activates the display page of the viewer 11 showing the electrocardiographic tracing with top priority and in real time.

The microprocessor 10 is programmed to activate the alarm signal SA (and consequently the displaying of the electrocardiographic tracing on the viewer 11) when an anomaly in the signal SE is detected: for example, if the anomaly is connected with an extrasystole, the alarm signal is triggered only on detection of a number K of extrasystoles (five, for example) in the preset unit of time (preferably one minute) considered to be an indicator of a pathology, and the alarm signal will appear on the viewer as EXTRA, as shown in Fig. 2. Obviously, the processing unit is programmed to recognize the different types of anomalous events that can be detected in the electrocardiographic signal such as, for example, extrasystoles, as already mentioned, ventricular tachycardia, asystole and ventricular fibrillation. The type of event is displayed on the viewer 11 (see also Fig. 5) by the abbreviated messages EXTRA (for extrasystole), TACHI (for tachycardia), ASI (for asystole) and FV (for ventricular fibrillation): the dental surgeon therefore knows immediately what type of anomaly has occurred.

As shown in Fig. 2, the alarm signal SA can be switched off (using appropriate means 30, also located on the console 23) under special circumstances, of a pathological nature, for example, such that the patient's cardiac activity may be anomalous for the system but may indicate the patient's normal heart condition.

The system disclosed will now be described from the point of view of its practical operation, starting from a situation in which the dental unit is switched off.

After seating the patient P on the chair 3 of the dental unit 7, the dental surgeon applies the three sensors 8a, 8b and 8c to the patient's left wrist and right-hand side of the neck and switches on the heart function monitoring system. For a few moments, the microprocessor analyses the applied signal SE and calibrates itself on the basis of the parameters of the signal, at least as regards the patient's heart rate and QRS traces (see Fig. 6) so as to define the reference signal SR with which to compare the processed signal SE from the patient during the treatment session.

Once it is calibrated, the system is ready and the surgeon can set the viewer 11 to display the functions required to proceed with the session.

During the session (see Fig. 6 again), the signal SE is acquired by the processing unit 21 which applies it to the first free memory bank 16, whilst the microprocessor 10 compares SE with the reference signal SR acquired initially.

If the signal SE is considered normal (see Fig. 6, right-hand path) the signal passes through the bank 16 without being stored. Instead, if the comparison between the signals SE and SR gives an anomalous result (see Fig. 6, left-hand path), the microprocessor 10 activates the alarm signal SA, sends a corresponding signal to activate the viewer 11 and causes the portion of tracing SE where the heart anomaly was detected to be fixed on the memory bank 16 and the next processed signal SE to be passed to the next memory bank 16. Obviously, as it compares the two signals, the microprocessor 10 also identifies the type of anomaly so that it can display on the viewer 11 the message describing the event.

The instant the viewer 11 is activated, the surgeon can read off the viewer itself the tracing of the signal SE processed at that moment, the message describing the type of event that has just occurred (see Fig. 2) and the heart rate updated to that moment. In order to view the tracing of the signal SE in which the anomalous event occurred, the surgeon must press a pushbutton 24 located on the console 23 to enter a submenu of the processing unit 21 which displays the last anomalous event recorded on the memory bank 16. From this submenu (see Fig. 5), the surgeon may also "navigate" in the memory banks using the aforesaid selection means 22 in order to view previous anomalous events or to return to subsequent ones, if any.

For example, if K extrasystoles are detected in the tracing of SE in one minute, the audible alarm signal is activated and the viewer 11 presents the display page, illustrated in Fig. 2, with the message EXTRA. From this moment on, it is the surgeon who decides whether to view the anomalous event (as mentioned earlier) or cancel the alarm display and to proceed with the operation by reactivating the display pages showing instrument data. Obviously, this procedure also applies to the other types of events.

In the case concerned, the processing unit 21 may have built-in compensation means (see Fig. 4) designed to allow the microprocessor 10 to keep the processed signal SE within a defined digital range so as to obtain a clear signal at all times, even when the patient P moves or is touched by the surgeon. For more convenience, the compensation means may be built into the microprocessor 10.

The capacity of the microprocessor 10 to discriminate normal signals from anomalous ones may be enhanced by programming the microprocessor 10 itself so that the reference signal SR is updated both as heart rate and as QRS tracing (see Fig. 6, block drawn with a broken line) each time a portion of cardiac tracing is stored in the memory bank 16. The system described therefore achieves the abovementioned aims thanks to the simple and practical arrangement of its components which allow the surgeon to operate on the patient without being distracted or interrupted, issuing an alarm signal if, and only if, it detects anomalous events in the patient's cardiac activity and recording the parts of the tracing concerned.

The patient is not in any way disturbed by the sensors which can be applied without having to remove any article of clothing and which allow the patient to move freely towards the left-hand side of the dental unit in order to pick up a rinsing tumbler 27 and to lean towards a cuspidor 28 (shown in Fig. 1). Nor do the sensors hamper the movements of the surgeon around the dental unit

The types of anomalous events listed above are the critical ones that must be recognized by the signal processing unit but obviously the unit can be programmed beforehand to recognize any type of anomalous event which the surgeon deems necessary. The invention described can be subject to modifications and variations without thereby departing from the scope of the inventive concept as defined in the appended claims.

## Claims

1. A heart function monitoring system applied to a dental unit consisting of a base (1) supporting a first column (2), placed beside a chair (3), and a first main holder (4) for dental instruments (5); said dental unit (7) being also provided with a plurality of sensors (8) designed to detect an electrocardiographic signal (SE), adapted to be applied to parts of the body of a patient (P) and forming part of an electrocardiograph (9); the dental unit (7) also being monitored by a processing unit or microprocessor (10) which also activates a viewer (11) for continuously displaying the operating function data and the accessory function data of the aforestated instruments, said monitoring system being **characterized in that** it comprises
- means (13) for the analog/digital conversion of said electrocardiographic signal (SE), the input of said means being connected to the sensors (8) and the output to the microprocessor (10) which processes the signal;
- storage means (14) designed to store the processed electrocardiographic signal (SE);
- means (10) for comparing the processed electrocardiographic input signal (SE) with a previously stored reference signal (SR) and which are designed to generate an alarm signal (SA) and a corresponding display on the viewer (11) if the signal (SE) is anomalous or differs in any way from the reference signal (SR);
and a control unit (17) of the viewer (11) capable of automatically activating the display page showing the electrocardiographic signal (SE), upon detection of an anomalous event, and disabling the display pages active at the moment and showing the data related to the instruments (5).

2. The system according to claim 1, **characterized in that** the said storage means (14) consist of a unit connected to the microprocessor (10) and memory banks (16) designed to receive and store a plurality of individual tracings of the processed electrocardiographic signal (SE) made in defined units of time depending on the storage capacity of the banks (16), sequentially, in first in first out fashion, in such a way that the signals stored are always the most recent; the system further comprising selection means (22) acting directly on the microprocessor (10) to display the contents of any of the said memory banks (16) on the viewer (11).

3. The system according to claim 2, **characterized in that** said storage means (14) receive and record in the memory banks (16) pluralities of individual tracings of the electrocardiographic signal (SE) when the latter is anomalous.

4. The system according to claim 1, **characterized in that** said microprocessor(10) processes the reference signal (SR) when the electrocardiograph (9) is switched on and updates the reference signal (SR) at regular intervals at least for heart rate.

5. The- system according to claim 1, **characterized in that** said microprocessor (10) activates the alarm signal (SA) and the corresponding display page when an anomaly in the signal (SE) is detected at least twice in a given unit of time.

6. The system according to claim 1, **characterized in that** said microprocessor (10) activates the alarm signal (SA) when the anomaly detected in the signal (SE) is an extrasystole repeated for at least a number of times (K) in a preset unit of time.

7. The system according to claim 1, **characterized in that** the sensors (8) are at least three in number (8a, 8b, 8c) of which two (8a, 8b) are input sensors adapted to be applied, respectively, to the left wrist and right-hand side of the neck of the patient (P), while the third sensor (8c) is neutral, is adapted to be applied near the first sensor (8a), that is to say, on the left wrist, and is designed to filter the signal measured by the other two sensors.

8. The system according to claim 1, **characterized in that** the sensors (8) are at least three in number (8a, 8b, 8c) of which two (8a, 8b) are input sensors adapted to be applied, respectively, to the left wrist and right ear lobe of the patient (P), while the third sensor (8c) is neutral, is adapted to be applied near the first sensor (8a), that is to say, on the left wrist, and is designed to filter the signal measured by the other two sensors.

9. The system according to claim 1, **characterized in that** the microprocessor (10) has compensation means designed to allow the microprocessor (10) to keep the processed signal (SE) within a defined digital range so as to obtain a clear signal even when the patient (P) moves or is touched.

## Patentansprüche

1. System zur Herzfunktionsüberwachung, angebracht an einer Dentaleinheit, die aus einer Basis (1) besteht, welche eine erste, neben einem Stuhl (3) angeordnete Säule (2) und eine erste Haupthalterung (4) für zahnärztliche Instrumente (5) trägt; wobei mit der genannten Dentaleinheit (7) auch eine Anzahl von Teil eines Elektrokardiographen (9) bildenden Fühlern (8) vorgesehen ist, dazu bestimmt, ein elektrokardiographisches Signal (SE) zu erfassen, und geeignet, an Körperteilen eines Patienten (P) angebracht zu werden; wobei die Dentaleinheit (7) ebenfalls von einer Prozessoreinheit oder einem Mikroprozessor (10) überwacht wird, welcher auch ein Sichtgerät (11) zur kontinuierlichen Anzeige der Betriebsfunktionsdaten und der Hilfsfunktionsdaten der vorgenannten Instrumente aktiviert, und wobei das genannte Überwachungssystem **dadurch gekennzeichnet ist, dass** es wie folgt enthält:
- Mittel (13) zur analog-digitalen Umwandlung der genannten elektrokardiographischen Signale (SE), wobei der Eingang der genannten Mittel an die Fühler (8) angeschlossen ist und der Ausgang an den Mikroprozessor (10), welcher das Signal verarbeitet;
- Speichermittel (14), die dazu bestimmt sind, das verarbeitete elektrokardiographische Signal (SE) zu speichern;
- Mittel (10) zum Vergleichen des elektrokardiographischen Eingangssignals (SE) mit einem vorher gespeicherten Bezugssignal (SR), und welche dazu bestimmt sind, ein Alarmsignal (SA) und eine entsprechende Anzeige auf dem Sichtgerät (11) zu erzeugen, wenn das Signal (SE) anomal ist oder sich in irgendeiner Weise von dem Bezugssignal (SR) unterscheidet; und
- eine Steuereinheit (17) des Sichtgerätes (11), in der Lage, die das elektrokardiographische Signal (SE) anzeigende Displayseite nach dem Erfassen eines anomalen Ereignisses automatisch zu aktivieren und die in dem Augenblick aktiven und die sich auf die Instrumente (5) beziehenden Daten anzeigenden Displayseiten zu entaktivieren.

2. System nach Patentanspruch 1, **dadurch gekennzeichnet, dass** die genannten Speichermittel (14) aus einer an den Mikroprozessor (10) und die Speicherbanken (16) angeschlossenen Einheit bestehen, dazu bestimmt, eine Anzahl von einzelnen Aufzeichnungen des verarbeiteten elektrokardiographischen Signals (SE) zu empfangen und zu speichern, die in festgelegten und von der Speicherkapazität der Banken (16) abhängigen Zeiteinheiten erzeugt wurden, aufeinanderfolgend und nach dem FIFO-Prinzip auf solche Weise, dass die gespeicherten Signale immer die neuesten sind; wobei das System weiterhin Wählmittel (22) enthält, welche direkt auf den Mikroprozessor (10) wirken, um den Inhalt einer jeden der genannten Speicherbanken (16) auf dem Sichtgerät (11) anzuzeigen.

3. System nach Patentanspruch 2, **dadurch gekennzeichnet, dass** die genannten Speichermittel (14) Anzahlen von einzelnen Aufzeichnungen des elektrokardiographischen Signals (SE) empfangen und in den Speicherbanken (16) speichern, wenn letzteres anomal ist.

4. System nach Patentanspruch 1, **dadurch gekennzeichnet, dass** der genannte Mikroprozessor (10) das Bezugssignal (SR) verarbeitet, wenn der Elektrokardiograph (9) eingeschaltet ist, und das Bezugssignal (SR) in gleichmässigen Intervallen und wenigstens die Herzfrequenz betreffend aktualisiert.

5. System nach Patentanspruch 1, **dadurch gekennzeichnet, dass** der genannte Mikroprozessor (10) das Alarmsignal (SA) und die entsprechende Displayseite aktiviert, wenn in einer bestimmten Zeiteinheit wenigstens zweimal eine Anomalie in dem Signal (SE) erfasst worden ist.

6. System nach Patentanspruch 1, **dadurch gekennzeichnet, dass** der genannte Mikroprozessor (10) das Alarmsignal (SA) aktiviert, wenn die in dem Signal (SE) erfasste Anomalie eine Extra-Systole ist, die sich wenigstens um eine Zahl von Malen (K) in einer bestimmten Zeiteinheit wiederholt.

7. System nach Patentanspruch 1, **dadurch gekennzeichnet, dass** die Fühler (8) wenigstens drei (8a, 8b, 8c) in der Zahl sind, von denen zwei (8a, 8b) Eingangsfühler sind, geeignet, jeweils am linken Handgelenk und an der rechten Halsseite des Patienten (P) angebracht zu werden, während der dritte Fühler (8c) neutral und geeignet ist, neben dem ersten Fühler (8a) angebracht zu werden, das heisst am linken Handgelenk, und dazu bestimmt, das durch die anderen beiden Fühler gemessene Signal zu filtern.

8. System nach Patentanspruch 1, **dadurch gekennzeichnet, dass** die Fühler (8) wenigstens drei (8a, 8b, 8c) in der Zahl sind, von denen zwei (8a, 8b) Eingangsfühler sind, geeignet, jeweils am linken Handgelenk und am rechten Ohrläppchen des Patienten (P) angebracht zu werden, während der dritte Fühler (8c) neutral und geeignet ist, neben dem ersten Fühler (8a) angebracht zu werden, das heisst am linken Handgelenk, und dazu bestimmt, das durch die anderen beiden Fühler gemessene Signal zu filtern.

9. System nach Patentanspruch 1, **dadurch gekennzeichnet, dass** der Mikroprozessor (10) Kompensationsmittel hat, dazu bestimmt, es dem Mikroprozessor (10) zu erlauben, das verarbeitete Signal (SE) innerhalb eines bestimmten digitalen Bereiches zu halten, so dass ein klares Signal erhalten wird, auch wenn sich der Patient (P) bewegt oder berührt wird.

## Revendications

1. Dispositif de surveillance de la fonction cardiaque appliqué sur un appareil dentaire, consistant en une base (1) supportant une première colonne (2), située à côté d'un fauteuil (3) et un premier support principal (4) pour des instruments dentaires (5) ; ladite unité dentaire (7) étant également équipée d'une pluralité de capteurs (8) prévus pour la détection d'un signal électrocardiographique (SE), pouvant être appliqués sur le corps d'un patient (P) et faisant partie d'un électrocardiographe (9) ; l'unité dentaire (7) étant contrôlée par une unité de traitement ou microprocesseur (10) qui active également un afficheur (11) pour la visualisation continue des données des fonctions opérationnelles et des fonctions accessoires desdits instruments dentaires, ledit dispositif de surveillance étant **caractérisé en ce qu'**il comprend :
- des moyens (13) pour la conversion analogique-numérique dudit signal électrocardiographique (SE), connectés en entrée à des capteurs (8) et en sortie au microprocesseur (10) de traitement du signal ;
- un système de mémoire (14) conçu pour enregistrer le signal électrocardiographique traité (SE) ;
- des moyens (10) pour comparer le signal électrocardiographique traité (SE) reçu avec un signal de référence (SR) précédemment enregistré, et conçus pour générer un signal d'alarme (SA) et visualiser un message correspondant sur l'afficheur (11) si le signal (SE) est anormal ou qu'il diffère en quoi que ce soit du signal de référence (SR) ; et
- une unité de commande (17) de l'afficheur (11), capable d'activer automatiquement la page-écran représentant le signal électrocardiographique (SE) après la détection d'un événement anormal et de fermer simultanément les pages-écrans montrant les données associées aux instruments (5) et qui étaient affichées à cet instant.

2. Le dispositif de surveillance selon la revendication 1, **caractérisé en ce que** ledit système de mémoire (14) consiste en une unité connectée au microprocesseur (10) et des blocs de mémoire (16) conçus pour recevoir et enregistrer une pluralité de tracés individuels du signal électrocardiographique (SE) traité, réalisés dans des unités de temps définies selon la capacité de stockage des blocs de mémoire (16), et ce de manière séquentielle selon la règle du premier entré premier sorti, de telle sorte que les signaux enregistrés soient toujours les plus récents ; ledit dispositif comprenant également des moyens de sélection (22), agissant directement sur le microprocesseur (10) pour visualiser le contenu de l'un quelconque des blocs de mémoire (16) sur l'afficheur (11).

3. Le dispositif de surveillance selon la revendication 2, **caractérisé en ce que** ledit système de mémoire (14) reçoit et enregistre dans les blocs de mémoire (16) des pluralités de tracés individuels du signal électrocardiographique (SE) lorsque ce dernier est anormal.

4. Le dispositif de surveillance selon la revendication 1, **caractérisé en ce que** ledit microprocesseur (10) traite le signal de référence (SR) à la mise sous tension de l'électrocardiographe (9) et actualise le signal de référence (SR) à intervalles réguliers, au moins en ce qui concerne la fréquence cardiaque.

5. Le dispositif de surveillance selon la revendication 1, **caractérisé en ce que** ledit microprocesseur (10) active le signal d'alarme (SA) et la page-écran correspondante si une anomalie est détectée au moins deux fois dans le signal (SE) au cours de l'unité de temps donnée.

6. Le dispositif de surveillance selon la revendication 1, **caractérisé en ce que** ledit microprocesseur (10) active le signal d'alarme (SA) lorsque l'anomalie détectée dans le signal (SE) est une extrasystole qui s'est répétée au moins un nombre de fois (K) dans une unité de temps donnée.

7. Le dispositif de surveillance selon la revendication 1, **caractérisé en ce que** lesdits capteurs (8) sont au moins au nombre de trois (8a, 8b, 8c), dont deux (8a, 8b) sont des capteurs d'entrée conçus pour être respectivement appliqués sur le poignet gauche et le côté droit du cou du patient (P), tandis que le troisième capteur (8c) est neutre, conçu pour être appliqué près du premier capteur (8a), c'est-à-dire sur le poignet gauche du patient, et servant au filtrage du signal mesuré par les deux autres capteurs.

8. Le dispositif de surveillance selon la revendication 1, **caractérisé en ce que** lesdits capteurs (8) sont au moins au nombre de trois (8a, 8b, 8c), dont deux (8a, 8b) sont des capteurs d'entrée conçus pour être respectivement appliqués sur le poignet gauche et le lobe de l'oreille droite du patient (P), tandis que le troisième capteur (8c) est neutre, conçu pour être appliqué près du premier capteur (8a), c'est-à-dire sur le poignet gauche du patient, et servant au filtrage du signal mesuré par les deux autres capteurs.

9. Le dispositif de surveillance selon la revendication 1, **caractérisé en ce que** ledit microprocesseur (10) est doté de moyens de compensation conçus pour permettre au microprocesseur (10) de maintenir le signal traité (SE) à l'intérieur d'une plage numérique définie, de manière à obtenir un signal clair même quand le patient (P) effectue des mouvements ou est touché.
